# FASCICULE DE BREVET EUROPEEN

(11) **EP 0 658 557 B1**
(45) Date de publication et mention de la délivrance du brevet: **02.09.1998**
(21) Numéro de dépôt: 94402862.0
(22) Date de dépôt: 13.12.1994
(51) Int. Cl.: C07D 471/22, A61K 31/495, A61K 31/435

(54) **Nouveaux analogues de l'éburnane, leur procédé de préparation et les compositions pharmaceutiques qui les contiennent**
Eburnan-Derivate, Verfahren zur ihrer Herstellung und diese enthaltende pharmazeutische Zusammensetzungen
Eburnane derivatives, processes for their preparation and pharmaceutical compositions containing them

(30) Priorité: 14.12.1993 FR 9314944
(43) Date de publication de la demande: 21.06.1995
(73) Titulaire: ADIR ET COMPAGNIE, 92415 Courbevoie Cédex (FR)
(72) Inventeur: Brion, Jean-Daniel, F-95320 Saint-leu la Forêt (FR); Thal, Claude, F-92330 Sceaux (FR); Demuynck, Luc, F-45000 Orléans (FR); Parmentier, Jean-Gilles, F-92130 Issy les Moulineaux (FR); Lepagnol, Jean, F-28210 Chaudon (FR); Lestage, Pierre, F-78170 La-Celle-Saint-Cloud (FR); Pujol, Jean-François, F-69002 Lyon (FR); Schmitt, Pascal, F-57070 Metz-Queleu (FR); Potier, Pierre, F-75007 Paris (FR)

(56) Documents cités:
- EP-A- 0 287 468
- WO-A-79/00319
- TETRAHEDRON, (INCL. TETRAHEDRON REPORTS), vol.44, no.7, 1988, OXFORD GB pages 1953 - 1958 R.Z. ANDRIAMIALISOA ET AL. '(.+-.)-14,15-dehydro-17-noreburnamonine and (.+-.)-2,7-dihydro-17-noreburnamonine.'

## Description

La présente invention concerne de nouveaux analogues de l'éburnane, leur procédé de préparation et les compositions pharmaceutiques qui les contiennent.

La littérature fournit de nombreux exemples de composés possédant une structure éburnane. C'est le cas notamment du brevet US-A-3,454,583 traitant de la vincamine ((3α,14β,16α)-(14,15-dihydro-14-hydroxy-éburnaménine-14-carboxylate de méthyle) et de ses dérivés pour leurs propriétés vasodilatatrices. Les demandes FR-A-2433528 et FR-A-2381048 présentent de nouveaux dérivés de 20,21-dinoréburnaménine et la demande EP-A-287468, de nouveaux dérivés de 17-aza-20,21-dinoréburnaménine.
Le brevet WO 7900319 décrit des structures indolo[2,3-a]quinolizidines possédant une action favorable dans le domaine de l'irrigation cérébrale, sans avoir les propriétés vasodilatatrices et antihypertensives des composés cités dans les brevets FR 2292475 et Fr 2315277.

Les composés de la présente invention se distinguent de ceux de l'art antérieur par des modifications apportées sur le cycle contenant les atomes 14 et 15 du squelette éburnane.

Outre leur structure nouvelle, la demanderesse a découvert que les composés de l'invention sont doués de propriétés pharmacologiques très intéressantes. Ils se révèlent notamment être de puissants inducteurs de tyrosine hydroxylase, de manière sélective ou non.

La présente invention concerne plus particulièrement les composés de formule générale (I) : 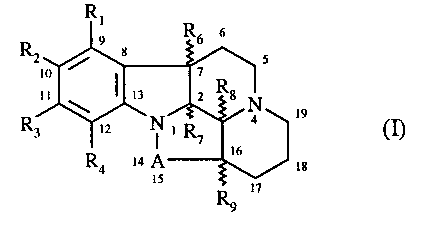 dans laquelle :
- R₁, R₂, R₃, R₄, identiques ou différents sont :
   * choisis indépendamment l'un de l'autre parmi :
      - un atome d'hydrogène,
      - un atome d'halogène,
      - un radical hydroxy,
      - un radical alkyle (C₁-C₆) linéaire ou ramifié éventuellement substitué par un ou plusieurs atomes d'halogène, par un ou plusieurs groupements amino, par un ou plusieurs groupements nitro, par un ou plusieurs groupements alkoxy (C₁-C₆) linéaire ou ramifiés ou par un ou plusieurs radicaux aryles, choisis parmi phényle et naphtyle, eux-mêmes éventuellement substitués par un ou plusieurs atomes d'halogène, groupements nitro ou amino, radicaux alkyles (C₁-C₆) ou alkoxy (C₁-C₆),
      - et un groupement alkoxy (C₁-C₆) linéaire ou ramifié éventuellement substitué par un ou plusieurs atomes d'halogène, par un ou plusieurs groupements amino, par un ou plusieurs groupements nitro, ou par un ou plusieurs groupements alkoxy (C₁-C₆) linéaire ou ramifiés,
   * ou bien R₁, R₂, R₃, et R₄, pris deux à deux et portés par des atomes de carbone adjacents, forment un groupement méthylènedioxy ou éthylènedioxy,
- R₆ et R₇ :
   . soit représentent chacun simultanément l'hydrogène, adoptant une configuration *cis* l'un par rapport à l'autre,
   . soit forment ensemble une liaison,
- R₈ et R₉ :
   . soit représentent chacun simultanément l'hydrogène, adoptant une configuration *cis* ou *trans* l'un par rapport à l'autre,
   . soit forment ensemble une liaison dans le cas où R₆ et R₇ forment également ensemble une double liaison,
- 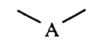 représente un radical bivalent choisi parmi 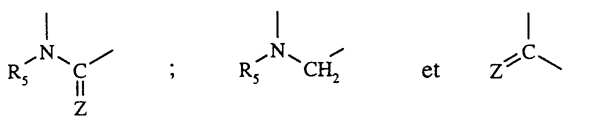
- Z est choisi parmi l'oxygène et le soufre,
- R₅ est choisi parmi l'hydrogène et un groupement alkyle (C₁-C₆) linéaire ou ramifié éventuellement substitué par un ou plusieurs :
   - atomes d'halogène,
   - radicaux alkoxy (C₁-C₆) linéaires ou ramifiés,
   - radicaux phényles éventuellement substitués par un ou plusieurs atomes d'halogènes, radicaux alkyles (C₁-C₆) linéaires ou ramifiés, ou alkoxy (C₁-C₆) linéaires ou ramifiés, ces radicaux alkyles et alkoxy étant éventuellement substitués par un ou plusieurs radicaux aryles, choisis parmi phenyle et naphtyle, substitués ou non par un ou plusieurs groupements choisis parmi les halogènes, les radicaux amino, nitro, alkyles (C₁-C₆) et alkoxy (C₁-C₆),
   - ou radicaux 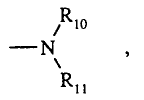 où R₁₀ et R₁₁ sont choisis indépendamment l'un de l'autre parmi l'hydrogène, les radicaux alkyles (C₁-C₆) linéaires ou ramifiés et les radicaux alkoxy (C₁-C₆) linéaires ou ramifiés,
   leurs éventuels N-oxydes, énantiomères et diastéréoisomères ainsi que, le cas échéant, leurs sels d'addition à un acide, pharmaceutiquement acceptables.

Parmi les acides utilisables pour former les sels pharmaceutiquement acceptables des composés de l'invention, on peut citer à titre non limitatif les acides chlorhydrique, bromhydrique, sulfurique, phosphorique et les acides acétique, propionique, maléique, fumarique, tartrique, nitrique, oxalique, benzoïque, méthanesulfonique, iséthionique et benzènesulfonique.

L'invention s'étend également au procédé de préparation des composés de formule (I), caractérisé en ce que le composé de formule (II) (dont la voie de synthèse est décrite par R.N. Schut et T.J. Leipzig. *J. Het. Chem.*, *3*, (1966), 101-102) : 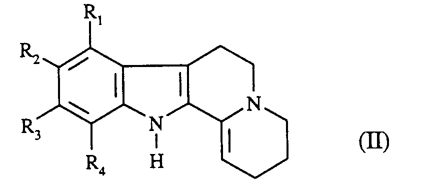
dans laquelle R₁, R₂, R₃ et R₄ sont tels que définis dans la formule (I),
est soumis à l'action d'un composé de formule (III) : 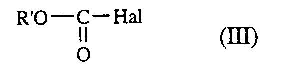
dans laquelle R' représente un groupement allyle contenant de 1 à 6 atomes de carbone en chaîne droite ou ramifiée, et Hal un atome d'halogène,
pour conduire au composé de formule (IV) : 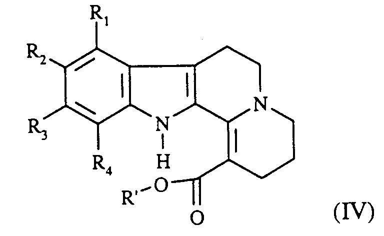
dans laquelle R₁, R₂, R₃, R₄ et R' sont tels que définis précédemment,
qui est ensuite hydrogéné en mélange d'énantiomères *cis* de formule (Va) : 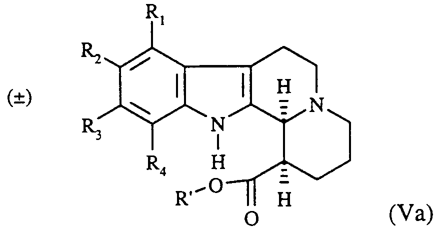
dans laquelle R₁, R₂, R₃, R₄ et R' sont tels que définis précédemment,
par action d'un agent d'hydrogénation, tel que le cyanoborohydrure de sodium, en milieu acide, par exemple acide acétique, et en solvant anhydre, tel que le tétrahydrofurane,
une réaction d'épimérisation pouvant être éventuellement réalisée, à froid, par exemple à 0°C, en solvant anhydre, comme le diméthoxyéthane, à l'aide d'un hydrure alcalin, tel que l'hydrure de sodium, afin d'obtenir le mélange d'énantiomères *trans* de formule (Vb) : 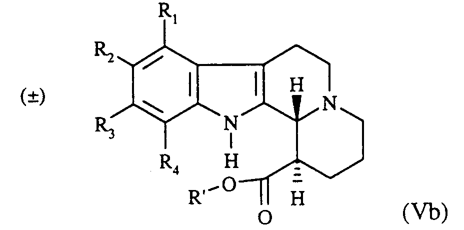
dans laquelle R₁, R₂, R₃, R₄ et R' sont tels que définis précédemment,
l'ensemble des composés de formules (Va) et (Vb) formant l'ensemble des composés de formule (V) : 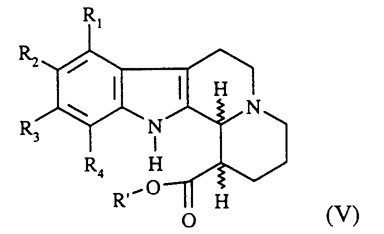
dans laquelle R₁, R₂, R₃, R₄ et R' sont tels que définis précédemment,
composés de formule (V) qui sont à nouveau soumis à hydrogénation, en milieu trifluoroacétique, par le cyanoborohydrure de sodium, pour conduire au composé de formule (VI) : 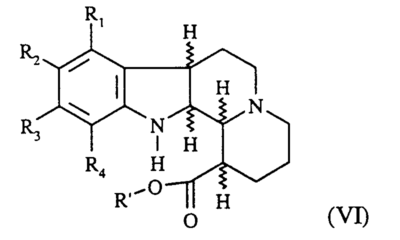
dans laquelle R₁, R₂, R₃, R₄ et R' sont tels que définis précédemment,
qui est soumis :
- soit: A/ à l'action d'un hydrure alcalin, l'hydrure de sodium par exemple, en solvant anhydre, tel que le tétrahydrofurane, à reflux, pour conduire au composé de formule (Xa) : 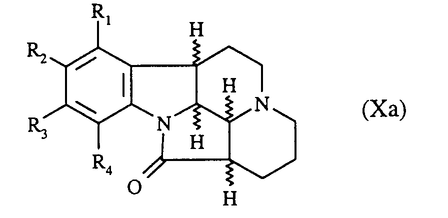
   dans laquelle R₁, R₂, R₃ et R₄ sont tels que définis précédemment,
- soit: B/ à l'action de nitrite de sodium, en milieu acide, acide acétique par exemple, puis en présence de zinc, pour conduire à l'hydrazine de formule (VII) : 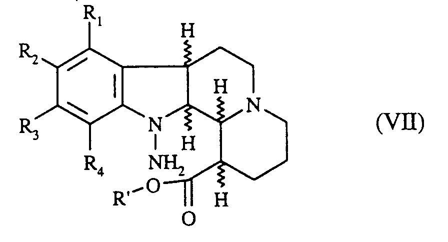
   dans laquelle R₁, R₂, R₃, R₄ et R' sont tels que définis précédemment,
qui est ensuite cyclisée, sous l'action d'un hydrure alcalin, l'hydrure de sodium par exemple, en composé de formule (VIII) : 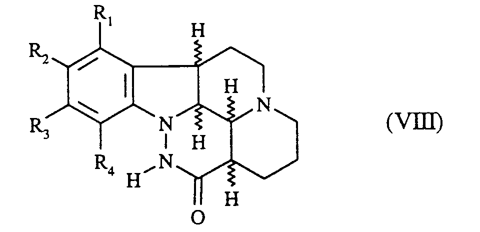
dans laquelle R₁, R₂, R₃ et R₄ sont tels que définis précédemment,
puis soumis, si on le souhaite, à l'action du composé de formule (IX) :

R'₅-Hal (IX)

dans laquelle R'₅ a les mêmes significations que le radical R₅, l'hydrogène excepté, et Hal représente un atome d'halogène,
après action d'un agent de déprotonation, le n-butyllithium ou l'hydrure de sodium par exemple, pour conduire au composé de formule (Xb) : 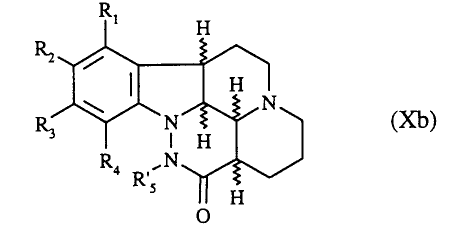
dans lesquelles R₁, R₂, R₃, R₄ et R'₅ sont tels que définis précédemment,
la fonction cétone de l'ensemble des composés de formules (VIII), (Xa) et (Xb) pouvant, si on le souhaite, être réduite, par exemple par de l'hydrure double de lithium et d'aluminium, en hydrocarbure correspondant, ou bien soumise à l'action du réactif de Lawesson, afin d'être transformée en la thiocétone correspondante,
l'ensemble des composés de formules (VIII), (Xa) et (Xb) ainsi que leurs éventuels produits de réductions ou leurs éventuels dérivés soufrés, formant l'ensemble des composés de formule (I/1) : 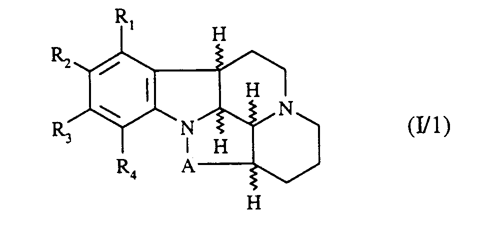
dans laquelle R₁, R₂, R₃, R₄ et A sont tels que définis précédemment,
qui sont :
- soit : soumis à l'action d'un oxydant doux, tel que le dioxyde de manganèse, pour conduire au composé de formule (I/2) : 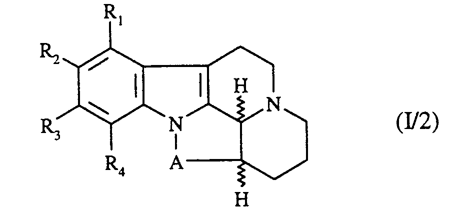
   dans laquelle R₁, R₂, R₃, R₄ et A sont tels que définis précédemment,
- soit : soumis à l'action d'un oxydant fort, tel que l'acide nitrique dans l'acide acétique, pour conduire au composé de formule (I/3) : 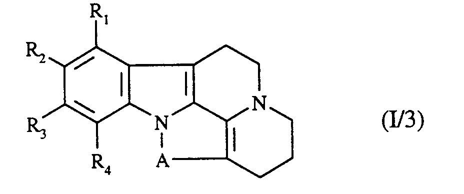
   dans laquelle R₁, R₂, R₃, R₄ et A sont tels que définis précédemment,
l'ensemble des composés de formules (I/1), (I/2) et (I/3) formant l'ensemble des composés de formule (I) qui sont, le cas échéant, purifiés par une technique classique de purification, séparés en leurs éventuels énantiomères et diastéréoisomères, par exemple par chromatographie sur colonne de type Chiralpak AD, et transformés en leurs N-oxydes ou sels d'addition à un acide, pharmaceutiquement acceptables.

Le composé de formule (V) peut également être préparé à partir du composé de formule (XI) : 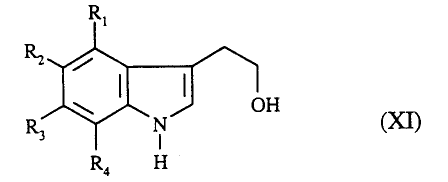
dans laquelle R₁, R₂, R₃, et R₄ sont tels que définis précédemment,
qui est transformé en composé de formule (XII) : 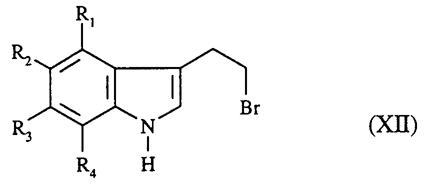
dans laquelle R₁, R₂, R₃, et R₄ sont tels que définis précédemment,
par action d'un agent de bromation, tel que le tétrabromure de carbone en présence de triphénylphosphine, ou le tribromure de phosphore,
que l'on fait réagir ensuite avec un composé de formule (XIII) : 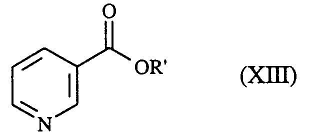
dans laquelle R' est tel que défini précédemment,
pour conduire, après hydrogénation catalytique, puis cyclisation en milieu acide, au composé de formule (V), tel que défini précédemment.

Les composés de formule (I) possèdent d'intéressantes propriétés pharmacologiques et notamment celle d'être de puissants inducteurs de tyrosine hydroxylase (TH). On sait que la tyrosine hydroxylase est une enzyme limitante qui contrôle particulièrement la synthèse du transmetteur dans les neurones catécholaminergiques et dopaminergiques centraux. La vitesse de synthèse de ce transmetteur est notamment reliée à l'apparition de dysfonctions toniques du cerveau que sont de nombreuses pathologies comportementales chez l'homme, telles que l'anxiété, les psychoses, les dépressions, etc...

Grâce à leur capacité d'induction de tyrosine hydroxylase, les composés de l'invention trouvent donc leur utilisation thérapeutique dans le traitement des dépressions, de l'anxiété, des troubles de la mémoire au cours de la sénescence et/ou de maladies dégénératives, et dans le traitement de la maladie de Parkinson.

La présente invention a également pour objet les compositions pharmaceutiques renfermant comme principe actif au moins un composé de formule générale (I), un de ses N-oxydes ou un de ses sels d'addition à un acide, pharmaceutiquement acceptables, seul ou en combinaison avec un ou plusieurs excipients ou véhicules inertes, non toxiques et appropriés.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par voie orale, rectale, intramusculaire ou parentérale.

La posologie peut varier notablement selon l'âge et le poids du patient, la voie d'administration, la nature de la maladie et les traitements associés et consiste en prises de 0,1 à 100 mg, une à cinq fois par jour.

Les exemples suivants illustrent l'invention sans toutefois la limiter en aucune façon. Les matières premières sont connues ou préparées à partir de modes opératoires connus.

### Préparation A : trans-9-Chloro-1,2,3,4,6,7,12,12b-octahydro-indolo-[2,3-a]quinolizine-1-carboxylate d'éthyle

### Etape A : 9-Chloro-2,3,4,6,7,12-hexahydro-indolo[2,3-a]quinolizine-1-carboxylate d'éthyle

18 g de 9-chloro-2,3,4,6,7,12-hexahydro-indolo[2,3-a]quinolizine, obtenus selon le procédé décrit dans le brevet FR-A-2315277, sont dissous dans 500 cm³ de toluène et 16,5 cm³ de di-isopropyléthylamine. L'ensemble est porté à 80°C pour ajouter ensuite, en 15 minutes, 11,4 g de chloroformiate d'éthyle. Le milieu réactionnel est alors maintenu à 80°C pendant 10 heures, pour être ensuite, après retour à température ambiante, chromatographié sur colonne de gel de silice (éluant: dichlorométhane), et conduire au produit attendu.
Rendement: 36 %
Point de fusion: 82-83°C

### Etape B : cis-9-Chloro-1,2,3,4,6,7,12,12b-octahydro-indolo[2,3-a]quinolizine-1-carboxylate d'éthyle

8,0 g du composé obtenu à l'étape précédente sont dissous dans 200 cm³ de tétrahydrofurane anhydre et 8 cm³ d'acide acétique.
L'ensemble est refroidi à environ 0°C-5°C par un bain de glace. 1,7 g de cyanoborohydrure de sodium est alors ajouté, sous argon, en 15 minutes.
Après 20 minutes d'agitation à température ambiante, le milieu réactionnel est hydrolysé par ajout de 25 cm³ d'une solution aqueuse de carbonate de sodium à 5%. Le tétrahydrofurane est distillé sous vide et le milieu réactionnel repris par 100 cm³ de dichlorométhane et 100 cm³ d'eau. Après traitement habituel de la phase organique, le résidu huileux obtenu est chromatographié sur colonne de gel de silice (éluant: dichlorométhane/méthanol, 95:5) pour conduire au produit attendu.
Rendement: 90 %

### Etape C : trans-9-Chloro-1,2,3,4,6,7,12,12b-octahydro-indolo[2,3-a]quinolizine-1-carboxylate d'éthyle

7,2 g du composé obtenu au stade précédent sont dissous, sous argon, dans 250 cm³ de 1,2-diméthoxyéthane anhydre. Après refroidissement de cette solution par un bain de glace (0°C-5°C), 2,4 g d'hydrure de sodium à 97 % sont ajoutés par portions, en 15 minutes. Le milieu réactionnel est abandonné sous agitation à température ambiante pendant 6 heures, puis est hydrolysé par une solution d'acide chlorhydrique 4N, à une température de -10°C jusqu'à obtenir un pH de 8-9. Le solvant est alors distillé sous vide et le résidu est repris par 150 cm³ d'eau et 150 cm³ d'acétate d'éthyle. Le traitement habituel de la phase organique fournit une huile correspondant au produit attendu.
Rendement: 85 %

### Préparation B : Chlorure de trans-10-méthoxy-1-méthoxycarbonyl-1,2,3,4,6,7,12,12b-octahydro-indolo[2,3-a]quinolizinium

### Etape A : 3-(2-Bromoéthyl)-6-méthoxy-indole

### a) 2-(6-Méthoxyindol-3-yl)-2-oxoacétate d'éthyle

7,5 cm³ de chlorure d'oxalyle sont ajoutés goutte à goutte, en trente minutes, à une solution de 8,3 g de 6-méthoxy-indole, préparé selon le procédé décrit par P.L. Feldman et H. Rapoport, *Synthesis*, (1986), 736, dans 100 cm³ d'éther éthylique anhydre, sous argon.
Après deux heures d'agitation, le précipité formé est séparé par filtration puis mis en suspension dans 200 cm³ d'éthanol.
Après dix heures d'agitation, le produit est essoré et rincé par de l'éther éthylique.
Rendement: 68 %
Point de fusion : 207-208°C

### b) 3-(2-Bromoéthyl)-6-méthoxy-indole

9,2 g du composé obtenu dans a) sont ajoutés à 200 cm³ de tétrahydrofurane anhydre et 4,2 g d'hydrure double de lithium et d'aluminium, sous argon. Le milieu réactionnel est porté à reflux pendant 3 heures. Après retour à température ambiante, celui-ci est hydrolysé par du sulfate de sodium humide. Les sels minéraux sont éliminés par filtration, le filtrat est alors évaporé à sec sous vide.

6,5 g de l'huile résiduelle obtenue sont remis en solution avec 15 g de tétrabromure de carbone dans 250 cm³ d'acétonitrile. L'ensemble est refroidi à 0°C-5°C pour ajouter 11,0 g de triphénylphosphine. Après une heure d'agitation, le solvant est éliminé par distillation sous vide et le résidu chromatographié sur colonne de gel de silice (éluant : dichlorométhane/heptane, 1:1), pour conduire au produit attendu.
Rendement: 95 %
Point de fusion: 109°C

### Etape B : Chlorure de trans-10-méthoxy-1-méthoxycarbonyl-1,2,3,4,6,7,12,12b-octahydro-indolo[2,3-a]quinolizinium

Une solution de 8,5 g du composé obtenu à l'étape A, 4,67 g de nicotinate de méthyle et 250cm³ d'acétone est portée à reflux pendant 48 heures. Après essorage et rinçage, 7,0 g du précipité obtenu sont soumis à hydrogénation à l'aide de 1,5 g de palladium sur charbon à 10 % dans 200 cm³ de méthanol, en présence de 2,9 cm³ de triéthylamine.
Après filtration du catalyseur et concentration sous vide, le résidu est chromatographié sur colonne de gel de silice (éluant : dichloro-méthane/méthanol, 98:2). 4,0 g du composé ainsi obtenu sont agités à température ambiante pendant 1 heure dans 20 cm³ de méthanol chlorhydrique et 20 cm³ d'éther éthylique. Le précipité est recueilli, rincé à l'éther puis séché.
Rendement : 52 %
Point de fusion : 250°C

### EXEMPLE 1 : Chlorure de (2RS,7SR),(3RS,16RS)-10-chloro-15-oxo-2,7,14,15-tétrahydro-14-aza-20,21-dinoréburnaméninium

### Etape A : 9-Chloro-1,2,3,4,6,7,7a,12,12a,12b-décahydro-indolo[2,3-a]quinolizine-1-carboxylate d'éthyle

A une solution, sous argon, de 6 g du composé obtenu dans la préparation A dans 200 cm³ d'acide trifluoroacétique sont ajoutés 5 g de cyanoborohydrure de sodium par portions de 100 mg et en renouvelant à chaque fois l'atmosphère réactionnelle par de l'argon. L'ensemble est agité pendant 4 heures avant d'être hydrolysé par une solution d'hydroxyde de sodium 10 N, en refroidissant, jusqu'à obtenir un pH de 9. Le milieu réactionnel est extrait par 2 fois 150 cm³ d'éther éthylique. Après traitement habituel de la phase organique, l'huile résiduelle est portée 1 heure au reflux dans l'éthanol. Le produit brut est chromatographié sur colonne de gel de silice (éluant: chlorure de méthylène/éthanol, 99:1) pour conduire au composé attendu.
Rendement : 42 %
Point de fusion: 124°C

### Etape B : 12-Amino-9-chloro-1,2,3,4,6,7,7a,12,12a,12b-décahydro-indolo[2,3-a] quinolizine-1-carboxylate d'éthyle

6,0 g du composé obtenu précédemment sont dissous dans 50 cm³ d'acide acétique et 25 cm³ d'eau. L'ensemble est refroidi au bain de glace puis, 1,38 g de nitrite de sodium dans 15 cm³ d'eau sont ajoutés. L'agitation est maintenue pendant 30 minutes, à une température inférieure à 10°C. Le mélange réactionnel est alors alcalinisé par une solution d'ammoniaque à 28 % puis dilué par 200 cm³ d'éthanol.
15 g de carbonate d'ammonium et 10 g de zinc en poudre sont alors ajoutés. La suspension est agitée vigoureusement, à température inférieure à 10°C, pendant 3 heures. Après élimination des sels minéraux par filtration, le filtrat est repris au chlorure de méthylène. Après traitement habituel de la phase organique, le résidu est purifié sur colonne de gel de silice (éluant : chlorure de méthylène/méthanol, 99:1) pour conduire au produit attendu.
Rendement : 52 %
Point de fusion : 172-175°C

### Etape C : Chlorure de (2RS,7SR),(3RS,16RS)-10-chloro-15-oxo-2,7,14,15-tétrahydro-14-aza-20,21-dinoréburnaméninium

3 g du composé obtenu à l'étape B, dissous dans 100 cm³ de tétrahydrofurane anhydre, sont additionnés, sous argon, de 960 mg d'hydrure de sodium à 97 %. L'ensemble est porté à reflux pendant 3 heures, refroidi à température ambiante et hydrolysé par quelques gouttes d'eau.
Le solvant est distillé, et l'huile résiduelle extraite par 100 cm³ d'eau et 2 fois 100 cm³ de dichlorométhane. Le traitement de la phase organique fournit un produit brut qui est transformé en son chlorure.
Rendement : 68 %
Point de fusion : 267-268°C
Microanalyse élémentaire : C₁₆H₁₈ClN₃O, HCl Masse mol. : 340,26

| | C % | H % | N % | Cl % |
|---|---|---|---|---|
| calculé | 56,48 | 5,63 | 12,35 | 20,84 |
| trouvé | 56,08 | 5,61 | 12,15 | 20,90 |

### EXEMPLE 2: (2RS,7SR),(3RS,16RS)-10-Chloro-14-méthyl-2,7,14,15-tétrahydro-14-aza-20,21-dinoréburnaménin-15-one

890 mg du composé obtenu à l'exemple 1, dissous dans 50 cm³ de tétrahydrofurane anhydre, sont additionnés de 3 cm³ de n-butyllithium 1,6M en solution dans l'hexane, goutte à goutte, à -30°C et sous argon. Après 30 minutes d'agitation, 710 mg d'iodure de méthyle dans 2 cm³ de tétrahydrofurane anhydre sont ajoutés. La température est ramenée à 25°C en 1 heure et 30 minutes, l'agitation est ensuite maintenue pendant 2 heures à cette température.
Le milieu réactionnel est hydrolysé par 80 cm³ d'eau, la phase aqueuse extraite par de l'acétate d'éthyle. Le traitement habituel de la phase organique fournit le produit attendu qui est purifié par recristallisation dans le méthanol.
Rendement : 56,5 %
Point de fusion : 209-210°C
Microanalyse élémentaire : C₁₇H₂₀ClN₃O Masse mol. : 317,82

| | C % | H % | N % | Cl % |
|---|---|---|---|---|
| calculé | 64,25 | 6,34 | 13,22 | 11,16 |
| trouvé | 64,19 | 6,19 | 13,20 | 11,19 |

### EXEMPLE 3: (2RS,7SR),(3RS,16RS)-14-Benzyl-10-chloro-2,7,14,15-tétrahydro-14-aza-20,21-dinoréburnaménin-15-one

En procédant comme dans l'exemple 2, mais en remplaçant l'iodure de méthyle par le bromure de benzyle, et après une étape de purification par chromatographie sur silice en utilisant successivement comme éluant le dichlorométhane/méthanol (95:5) puis l'acétate d'éthyle, on obtient le composé attendu.
Rendement : 47 %
Point de fusion : 153-154°C
Microanalyse élémentaire : C₂₃H₂₄ClN₃O Masse mol.: 393,92

| | C % | H % | N % | Cl % |
|---|---|---|---|---|
| calculé | 70,13 | 6,14 | 10,67 | 9,00 |
| trouvé | 70,02 | 5,91 | 10,35 | 9,17 |

### EXEMPLE 4: Dichlorure de (2RS,7SR),(3RS,16RS)-10-chloro-2,7,14,15-tétrahydro-14-aza-20,21-dinoréburnaméninium

850 mg du composé obtenu à l'exemple 1 dans 50 cm³ de tétrahydrofurane anhydre sont additionnés de 850 mg d'hydrure double de lithium et d'aluminium. Le milieu réactionnel est porté à reflux pendant 3 heures. 40 cm³ d'eau saturée en chlorure de sodium sont alors ajoutés, goutte à goutte, dans le milieu réactionnel refroidi à 0°C.
Le mélange est extrait à l'acétate d'éthyle et le traitement habituel de la phase organique fournit un produit brut qui est transformé en son dichlorure, correspondant au produit attendu.
Rendement : 24 %
Point de fusion : 266-268°C
Microanalyse élémentaire : C₁₆H₂₀ClN₃, 2HCl Masse mol.: 362,73

| | C % | H % | N % |
|---|---|---|---|
| calculé | 52,98 | 6,11 | 11,58 |
| trouvé | 52,33 | 6,18 | 11,00 |

### EXEMPLE 5: (3RS,16RS)-10-Chloro-14,15-dihydro-14-aza-20,21-dinoréburnaménin-15-one

700 mg de dioxyde de manganèse sont ajoutés à 305 mg du composé obtenu à l'exemple 1, dans 25 cm³ de dichlorométhane. Le milieu réactionnel est agité 1 heure et 30 minutes à température ambiante puis filtré sur célite. Après évaporation du filtrat, purification et recristallisation dans le méthanol, le composé titre est obtenu.
Rendement : 40 %
Point de fusion : 252-254°C
Microanalyse élémentaire : C₁₆H₁₆ClN₃O Masse mol.: 301,78

| | C % | H % | N % |
|---|---|---|---|
| calculé | 63,68 | 5,34 | 13,92 |
| trouvé | 63,41 | 5,26 | 13,57 |

### EXEMPLES 6 A 14 :

En procédant de façon analogue à l'exemple 1, mais en remplaçant au stade A la 9-chloro-1,2,3,4,6,7,12,12b-octahydro-indolo[2,3-a]quinolizine-1-carboxylate d'éthyle par les composés de départ adéquats, les composés des exemples suivants sont obtenus :

### EXEMPLE 6: Chlorure de (2RS,7SR),(3RS,16RS)-15-oxo-2,7,14,15-tétrahydro-14-aza-20,21-dinoréburnaméninium

Point de fusion : > 250°C
Microanalyse élémentaire : C₁₆H₁₉N₃O, HCl Masse mol.: 305,81

| | C % | H % | N % |
|---|---|---|---|
| calculé | 62,84 | 6,59 | 13,74 |
| trouvé | 62,79 | 6,58 | 13,64 |

### EXEMPLE 7: Chlorure de (2RS,7SR),(3RS,16RS)-10-bromo-15-oxo-2,7,14,15-tétrahydro-14-aza-20,21-dinoréburnaméninium

Point de fusion : > 275°C
Microanalyse élémentaire : C₁₆H₁₈BrN₃O, HCl, 0,5CH₃OH Masse mol.: 400,73

| | C % | H % | N % | Cl % |
|---|---|---|---|---|
| calculé | 49,46 | 5,28 | 10,49 | 8,85 |
| trouvé | 49,28 | 5,13 | 10,14 | 8,39 |

### EXEMPLE 8: Chlorure de (2RS,7SR),(3RS,16RS)-10-méthoxy-15-oxo-2,7,14,15-tétrahydro-14-aza-20,21-dinoréburnaméninium

Point de fusion : 110-112°C
Microanalyse élémentaire : C₁₇H₂₁N₃O₂, HCl, 0,5CH₃OH Masse mol.: 351,93

| | C % | H % | N % | Cl % |
|---|---|---|---|---|
| calculé | 59,65 | 6,82 | 11,93 | 9,93 |
| trouvé | 59,25 | 6,88 | 11,29 | 9,62 |

### EXEMPLE 9: Chlorure de (2RS,7SR),(3RS,16RS)-11-méthoxy-15-oxo-2,7,14,15--tétrahydro14-aza-20,21-dinoréburnaméninium

Point de fusion : > 280°C
Microanalyse élémentaire : C₁₇H₂₁N₃O₂, HCl Masse mol.: 335,84

| | C % | H % | N % |
|---|---|---|---|
| calculé | 60,21 | 6,41 | 12,34 |
| trouvé | 60,80 | 6,60 | 12,51 |

### EXEMPLE 10: Chlorure de (2RS,7SR),(3RS,16RS)-10,11-diméthoxy-15-oxo-2,7,14,15-tétrahydro-14-aza-20,21-dinoréburnaméninium

Point de fusion : 282°C
Microanalyse élémentaire : C₁₈H₂₃N₃O₃, HCl Masse mol.: 365,90

| | C % | H % | N % |
|---|---|---|---|
| calculé | 59,09 | 6,61 | 11,49 |
| trouvé | 58,81 | 6,46 | 11,13 |

### EXEMPLE 11: (2RS,7SR),(3RS,16RS)-10-Trifluorométhoxy-2,7,14,15-tétrahydro-14-aza-20,21-dinoréburnaménin-15-one

Point de fusion : 74-75°C
Microanalyse élémentaire : C₁₇H₁₈F₃N₃O₂ Masse mol.: 353,35

| | C % | H % | N % |
|---|---|---|---|
| calculé | 57,78 | 5,13 | 11,89 |
| trouvé | 58,12 | 5,46 | 11,38 |

### EXEMPLE 12: Chlorure de (2RS,7SR),(3RS,16RS)-10,11-méthylènedioxy-15-oxo-2,7,14,15-tétrahydro-14-aza-20,21-dinoréburnaméninium

Point de fusion : 268-269°C
Microanalyse élémentaire : C₁₇H₁₉N₃O₃, HCl Masse mol.: 349,82

| | C % | H % | N % |
|---|---|---|---|
| calculé | 58,14 | 5,82 | 11,77 |
| trouvé | 57,99 | 6,00 | 11,48 |

### EXEMPLE 13: Chlorure de (2RS,7SR),(3RS,16RS)-10-méthyl-15-oxo-2,7,14,15-tétrahydro-14-aza-20,21-dinoréburnaméninium

Point de fusion : 280°C
Microanalyse élémentaire : C₁₇H₂₁N₃O, HCl, 0,5 CH₃OH Masse mol.: 335,84

| | C % | H % | N % | Cl % |
|---|---|---|---|---|
| calulé | 62,58 | 7,20 | 12,51 | 10,55 |
| trouvé | 62,47 | 7,37 | 11,85 | 10,32 |

### EXEMPLE 14: Chlorure de (2RS,7SR),(3RS,16RS)-11-méthyl-15-oxo-2,7,14,15-tétrahydro-14-aza-20,21-dinoréburnaméninium

Point de fusion : > 275°C
Microanalyse élémentaire : C₁₇H₂₁N₃O, HCl Masse mol.: 319,81

| | C % | H % | N % | Cl % |
|---|---|---|---|---|
| calculé | 63,85 | 6,93 | 13,14 | 11,08 |
| trouvé | 63,54 | 6,64 | 13,07 | 11,15 |

### EXEMPLE 15: (2RS,7SR),(3RS,16RS)-10-Chloro-2,7,14,15-tétrahydro-14-aza-20,21-dinoréburnaménin-15-thione

305 mg du composé obtenu à l'exemple 1 dans 50 cm³ de toluène anhydre sont mis en présence de 400 mg de réactif de Lawesson. L'ensemble est chauffé à reflux de toluène pendant 1 heure 30 minutes, puis hydrolysé par 50 cm³ d'hydroxyde de sodium 0,5 N. Le traitement habituel de la phase organique fournit le produit attendu.
Point de fusion : 194°C
Microanalyse élémentaire : C₁₆H₁₈ClN₃S Masse mol.: 319,86

| | C % | H % | N % | S % |
|---|---|---|---|---|
| calculé | 60,08 | 5,67 | 13,14 | 10,02 |
| trouvé | 59,93 | 5,78 | 13,00 | 9,98 |

### EXEMPLE 16: 14,15-Dihydro-3,16-didéshydro-14-aza-20,21-dinoréburnaménin-15-one

810 mg du composé obtenu à l'exemple 6 sont mis en solution dans un mélange constitué de 10 cm³ d'acide acétique et de 0,2 cm³ d'acide nitrique. Après 1 heure et 30 minutes d'agitation à +10°C, de l'ammoniaque est ajouté jusqu'à obtenir un pH d'une valeur d'environ 10. Le précipité formé est filtré, lavé à l'eau et séché sous vide avant d'être purifié par chromatographie sur colonne de gel de silice (éluant dichlorométhane méthanol, 97:3). 100 mg d'un solide jaune correspondant au produit attendu sont obtenus.
Rendement : 13 %
Point de fusion : 260°C (Décomposition)

### EXEMPLE 17: trans-(3RS,16RS)-14,15-Dihydro-14-aza-20,21-dinoréburnaménin-15-one

800 mg d'oxyde de manganèse sont ajoutés à 250 mg de composé de l'exemple 6 dans 25 cm³ de dichlorométhane. La réaction est conduite pendant 2 heures. Le milieu réactionnel est filtré sur célite. Le filtrat est évaporé et le résidu purifié par recristallisation dans l'éthanol pour fournir 170 mg de composé attendu.
Point de fuion : 254-255°C
Microanalyse élémentaire : C₁₆H₁₇N₃O Masse mol.: 267,33

| | C % | H % | N % |
|---|---|---|---|
| calculé | 71,89 | 6,41 | 15,72 |
| trouvé | 72,23 | 6,47 | 15,68 |

### EXEMPLE 18: Dichlorure de trans-(3RS,16RS)-14,15-dihydro-14-aza-20,21-dinoréburnaméninium

### Etape A : trans-(3RS,16RS)-14,15-Dihydro-14-aza-20,21-dinoréburnaménine

170 mg du composé obtenu à l'exemple 17 en solution dans 20 cm³ de tétrahydrofurane sont additionnés de 36 mg d'hydrure double de lithium et d'aluminium. Après 2 heures d'agitation, l'ensemble est hydrolysé, les sels minéraux filtrés et le filtrat évaporé à sec.
Point de fusion : 196°C
Microanalyse élémentaire : C₁₆H₁₉N₃ Masse mol.: 253,35

| | C % | H % | N % |
|---|---|---|---|
| calculé | 75,08 | 7,27 | 16,36 |
| trouvé | 75,85 | 7,56 | 16,59 |

### Etape B : Dichlorure de trans-(3RS,16RS)-14,15-dihydro-14-aza-20,21-dinoréburnaméninium

Le composé obtenu à l'étape précédente est transformé en son dichlorure dans l'éthanol chlorhydrique pour fournir 100 mg de composé attendu.
Rendement global : 57 %
Point de fusion : 239-240°C
Microanalyse élémentaire : C₁₆H₁₉N₃, 2HCl Masse mol.: 326,27

| | C % | H % | N % | Cl % |
|---|---|---|---|---|
| calculé | 58,90 | 6,49 | 12,88 | 21,73 |
| trouvé | 59,06 | 6,22 | 12,84 | 21,88 |

### EXEMPLE 19: Dichlorure de (2SR,7RS),(3RS,16RS)-2,7,14,15-tétrahydro-14-aza-20,21-dinoréburnaméninium

Procédé identique à celui décrit dans l'exemple 18 à partir du composé obtenu à l'exemple 6.
Rendement : 78 %
Point de fusion : > 275°C
Microanalyse élémentaire : C₁₆H₂₁N₃, 2HCl Masse mol.: 328,29

| | C % | H % | N % | Cl % |
|---|---|---|---|---|
| calculé | 58,54 | 7,06 | 12,80 | 21,60 |
| trouvé | 58,82 | 7,43 | 12,50 | 20,81 |

### EXEMPLE 20: (1aRS,12bRS),(7aSR,12aRS)-9-Chloro-1a,2,3,6,7,7a,12a,12b-octahydro-1H,4H-benzo[5,6]pyrrolizino[2,1,7-ija]quinolizin-1-one

A 250 mg de composé obtenu à l'étape A de l'exemple 1 dans 25 cm³ de tétrahydrofurane anhydre sont ajoutés, sous argon, 80 mg d'hydrure de sodium à 97 %. L'ensemble est porté à reflux pendant 3 heures, puis refroidi à température ambiante et hydrolysé par quelques gouttes d'eau. Le solvant est évaporé et le résidu est repris par 20 cm³ d'eau et deux fois 15 cm3 de dichlorométhane. Le traitement habituel de la phase organique fournit un produit brut, qui, recristallisé dans l'éther isopropylique correspond au produit attendu.
Rendement : 85 %
Point de fusion : 176-177°C
Microanalyse élémentaire : C₁₆H₁₇ClN₂O Masse mol.: 288,78

| | C % | H % | N % | Cl % |
|---|---|---|---|---|
| calculé | 66,55 | 5,93 | 9,70 | 12,28 |
| trouvé | 66,62 | 5,61 | 9,30 | 12,24 |

### EXEMPLE 21: (1aRS,12bRS),(7aSR,12aRS)-9-Méthyl-1a,2,3,6,7,7a,12a,12b-octahydro-1H,4H-benzo[5,6]pyrrolizino[2,1,7-ija]quinolizin-1-one

En procédant de la manière indiquée dans l'exemple précédent, à partir du composé obtenu dans la première étape de l'exemple 13, le produit attendu est obtenu.
Rendement : 78 %
Point de fusion : 152-153°C
Microanalyse élémentaire : C₁₇H₂₀N₂O Masse mol.: 268,36

| | C % | H % | N % |
|---|---|---|---|
| calculé | 76,09 | 7,51 | 10,44 |
| trouvé | 76,28 | 7,55 | 10,40 |

### EXEMPLE 22: (1aRS,12bRS),(7aSR,12aRS)-9-Méthoxy-1a,2,3,6,7,7a,12a,12b-octahydro-1H,4H-benzo[5,6]pyrrolizino[2,1,7-ija]quinolizin-1-one

En procédant comme décrit dans l'exemple 20, à partir du composé obtenu dans la première étape de l'exemple 8, le produit attendu est obtenu.
Rendement : 86 %
Point de fusion : 145-146°C
Microanalyse élémentaire : C₁₇H₂₀N₂O₂ Masse mol.: 284,36

| | C % | H % | N % |
|---|---|---|---|
| calculé | 71,81 | 7,09 | 9,85 |
| trouvé | 71,63 | 7,02 | 9,90 |

### EXEMPLE 23: (2RS,7SR),(3RS,16RS)-14-Benzyl-2,7,14,15-tétrahydro-14-aza-20,21-dinoréburnaménin-15-one

270 mg de composé obtenu à l'exemple 6, dans 10 cm³ de tétrahydrofurane anhydre sont additionnés, sous argon et à -30°C, de 1,2 cm³ de n-butyllithium 1,6 M dans l'hexane. Après 30 minutes d'agitation, 250 mg de bromure de benzyle sont ajoutés. Après retour à température ambiante (2 heures), le milieu réactionnel est maintenu 16 heures sous agitation, puis hydrolysé et extrait par de l'acétate d'éthyle. Le traitement habituel de la phase organique fournit un produit qui est purifié par chromatographie sur colonne de gel de silice (éluant : acétate d'éthyle) pour fournir 250 mg de composé attendu.
Rendement : 71 %
Point de fusion : 122°C
Microanalyse élémentaire : C₂₃H₂₅N₃O Masse mol.: 359,48

| | C % | H % | N % |
|---|---|---|---|
| calculé | 75,85 | 7,01 | 11,69 |
| trouvé | 75,26 | 6,88 | 11,05 |

### EXEMPLE 24: trans-(3RS,16RS)-14-Benzyl-14,15-dihydro-14-aza-20,21-dinoréburnaménin-15-one

A 100 mg de composé obtenu à l'exemple 17, dans 15 cm³ de tétrahydrofurane anhydre, chauffés à 50°C, sont ajoutés, sous argon, 50 mg d'hydrure de sodium à 97 %. Après 20 minutes de réaction, 170 mg de bromure de benzyle dans 2 cm³ de tétrahydrofurane sont ajoutés goutte à goutte. Après deux heures, le milieu réactionnel est refroidi, I'excès d'hydrure détruit par de l'eau et le mélange réactionnel extrait par de l'acétate d'éthyle. Le traitement de la phase organique fournit un produit brut qui est recristallisé dans le méthanol, pour conduire, après séchage, à 100 mg de composé attendu.
Rendement : 75%
Point de fusion : 158°C
Microanalyse élémentaire : C₂₃H₂₃N₃O Masse mol.: 357,46

| | C % | H % | N % |
|---|---|---|---|
| calculé | 77,28 | 6,49 | 11,76 |
| trouvé | 76,93 | 6,35 | 11,80 |

### ETUDE PHARMACOLOGIQUE

### EXEMPLE A: Etude de l'effet inducteur de tyrosine hydroxylase

Les animaux utilisés sont des rats mâles Sprague-Dawley (Iffa-Credo, France) pesant 220-250 g qui sont placés dans une chambre climatisée à 22°C avec un cycle lumière/obscurité 12h/12h. La nourriture comme l'eau sont fournis à volonté. Les animaux sont traités avec une dose unique (voie intrapéritonéale) de produit, les témoins recevant la solution véhicule. Pour l'étude de l'effet inducteur, les animaux sont sacrifiés 3 jours après l'injection.

Après sacrifice de l'animal par décapitation, le cerveau est rapidement prélevé puis congelé sur une plaque métallique refroidie à -80°C. La dissection des structures s'effectue sur des coupes sériées de 200 µm. Les cerveaux congelés sont coupés selon l'axe postéro-antérieur avec un microtome à platine réfrigéré. Les prélèvements des tissus se font à l'emporte-pièce grâce à un tube métallique de 2 mm de diamètre interne. Dans ces conditions, les structures sont prélevées en excès. Huit sections de 200 µm (4 pour le Locus postérieur, 4 pour le Locus antérieur) et huit coupes de 200 µm (pour la substance noire) sont obtenues selon l'axe postéro-antérieur. Afin d'améliorer la reproductibilité des prélèvements, ceux-ci ont été effectués après visualisation histochimique des structures par la méthode colorimétrique de Glenner (1957). Le principe de cette technique repose sur l'oxydation d'un substrat, la tryptamine, fourni aux coupes incubées, par la monoamine oxydase (MAO) endogène, qui intervient dans la dégradation des catécholamines. Le produit oxydé de la MAO réduit ensuite des sels de tétrazolium du bain d'incubation qui précipitent.

Les structures cérébrales sont placées dans un tampon phosphate (5mM, pH 6) contenant 0,2 % de Triton X-100 permettant d'obtenir une parfaite solubilisation des protéines, en particulier de la TH membranaire. L'extraction de la TH est obtenue avec 3 cycles de congélation/dé-congélation (-80°C/température ambiante) suivie d'une centrifugation à 10 000 g pendant 20 minutes à +4°C. La quantité de TH est mesurée par dosage immunoautoradiographique (méthode de "Dot Blot"). Brièvement, la protéine est déposée directement sur une feuille de nitrocellulose, saturée dans une solution de "bovine sérum albumine" 1 % (BSA) pendant 1/2 h et incubée toute la nuit dans une solution d'anticorps anti-TH monoclonal diluée au 1/3000. Le complexe est révélé après incubation pendant 2 heures dans une solution de protéine A radioactive (¹³⁵I) diluée au 1/1000. Après exposition de la nitrocellulose sur un film MP pendant 4-7 jours environ, le développement de ce dernier est réalisé dans des conditions standard. La quantification du signal obtenu pour chaque dépôt s'effectue par densitométrie à l'aide d'un analyseur d'image Imstar. La quantité de TH (exprimée en unités TH par structure) est calculée à partir d'une gamme de TH de surrénale standard.

Les résultats sont présentés dans le tableau I suivant :

**TABLEAU I**

| Mesure de la quantité de TH dans le Locus coeruleus antérieur (LCA) et postérieur (LCP) après administration i.p. (30 mg/kg) | | |
|---|---|---|
| **N° Ex** | **LCA** | **LCP** |
| 1 | +45%*** | +30%*** |
| 8 | +45%*** | +30%*** |
| 9 | +49%** | +19% (ns) ⁽¹⁾ |
| 13 | +15%** | +15%** |
| 14 | +44%** | +22% (ns) |
| 18 | +47%** | +28%* |
| 20 | +20% | 0% |
| 21 | +30%* | 0% |
| 22 | +40%* | 0% |

| | | |
|---|---|---|
| ⁽¹⁾ : +14%* dans la Substantia nigra | | |
| ns : non spécifique | | |
| * : p < 0,05 | | |
| ** : p < 0,01 | | |
| *** : p < 0,001 | | |

## Revendications

1. Composés de formule générale (I): 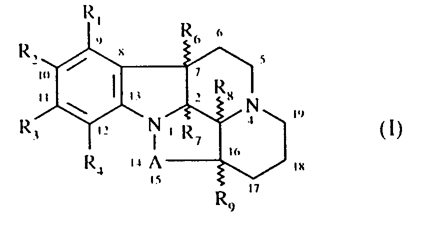 dans laquelle :
- R₁, R₂, R₃, R₄, identiques ou différents sont :
* choisis indépendamment l'un de l'autre parmi :
- un atome d'hydrogène,
- un atome d'halogène,
- un radical hydroxy,
- un radical alkyle (C₁-C₆) linéaire ou ramifié éventuellement substitué par un ou plusieurs atomes d'halogène, par un ou plusieurs groupements amino, par un ou plusieurs groupements nitro, par un ou plusieurs groupements alkoxy (C₁-C₆) linéaire ou ramifiés ou par un ou plusieurs radicaux aryles, choisis parmi phényle et naphtyle, eux-mêmes éventuellement substitués par un ou plusieurs atomes d'halogène, groupements nitro ou amino, radicaux alkyles (C₁-C₆) ou alkoxy (C₁-C₆),
- et un groupement alkoxy (C₁-C₆) linéaire ou ramifié éventuellement substitué par un ou plusieurs atomes d'halogène, par un ou plusieurs groupements amino, par un ou plusieurs groupements nitro, ou par un ou plusieurs groupements alkoxy (C₁-C₆) linéaire ou ramifiés,
* ou bien R₁, R₂, R₃, et R₄ pris deux à deux et portés par des atomes de carbone adjacents, forment un groupement méthylènedioxy ou éthylènedioxy,
- R₆ et R₇ :
. soit représentent chacun simultanément l'hydrogène, adoptant une configuration *cis* l'un par rapport à l'autre,
. soit forment ensemble une liaison,
- R₈ et R₉ :
. soit représentent chacun simultanément l'hydrogène, adoptant une configuration *cis* ou *trans* l'un par rapport à l'autre,
. soit forment ensemble une liaison, dans le cas où R₆ et R₇ forment également ensemble une double liaison
- 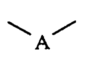 représente un radical bivalent choisi parmi 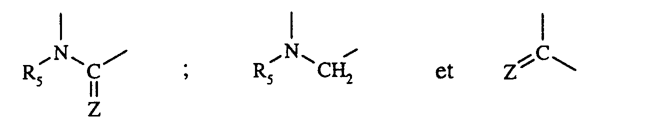
- Z est choisi parmi l'oxygène et le soufre,
- R₅ est choisi parmi l'hydrogène et un groupement alkyle (C₁-C₆) linéaire ou ramifié éventuellement substitué par un ou plusieurs :
- atomes d'halogène,
- radicaux alkoxy (C₁-C₆) linéaires ou ramifiés,
- radicaux phényles éventuellement substitués par un ou plusieurs atomes d'halogène, radicaux alkyles (C₁-C₆) linéaires ou ramifiés, ou alkoxy (C₁-C₆) linéaires ou ramifiés, ces radicaux alkyles et alkoxy étant éventuellement substitués par un ou plusieurs radicaux aryles, choisis parmi phényle et naphtyle, substitués ou non par un ou plusieurs groupements choisis parmi les halogènes, les radicaux amino, nitro, alkyles (C₁-C₆) et alkoxy (C₁-C₆),
- ou radicaux 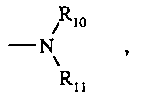 où R₁₀ et R₁₁ sont choisis indépendamment l'un de l'autre parmi l'hydrogène, les radicaux alkyles (C₁-C₆) linéaires ou ramifiés et les radicaux alkoxy (C₁-C₆) linéaires ou ramifiés,
leurs éventuels N-oxydes, énantiomères et diastéréoisomères ainsi que, le cas échéant, leurs sels d'addition à un acide, pharmaceutiquement acceptables.

2. Composé selon la revendication 1 qui est la 10-méthoxy-2,7,14,15-tétrahydro-14-aza-20,21-dinoréburnaménin-15-one,
ses N-oxydes, énantiomères et diastéréoisomères et sels d'addition à un acide, pharmaceutiquement acceptables.

3. Composé selon la revendication 1 qui est la 10-chloro-2,7,14,15-tétrahydro-14-aza-20,21-dinoréburnaménin-15-one,
ses N-oxydes, énantiomères et diastéréoisomères et sels d'addition à un acide, pharmaceutiquement acceptables.

4. Composé selon la revendication 1 qui est la 14,15-dihydro-14-aza-20,21-dinoréburnaménine,
ses N-oxydes, énantiomères et diastéréoisomères et sels d'addition à un acide, pharmaceutiquement acceptables.

5. Composé selon la revendication 1 qui est la 10,11-méthylènedioxy-2,7,14,15-tétrahydro-14-aza-20,21-dinoréburnaménin-15-one,
ses N-oxydes, énantiomères et diastéréoisomères et sels d'addition à un acide, pharmaceutiquement acceptables.

6. Composé selon la revendication 1 qui est la 9-méthoxy-1a,2,3,6,7,7a,12a,12b-octahydro-1*H*,5*H*-benzo[5,6]pyrrolizino[2,1,7-ija]quinolizin-1-one,
ses N-oxydes, énantiomères et diastéréoisomères et sels d'addition à un acide, pharmaceutiquement acceptables.

7. Procédé de préparation des composés de formule (I), caractérisé en ce que le composé de formule (II) : 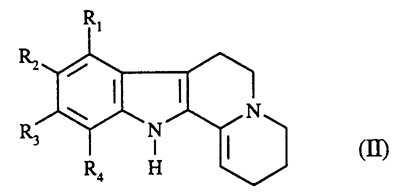
dans laquelle R₁, R₂, R₃ et R₄ sont tels que définis dans la formule (I),
est soumis à l'action d'un composé de formule (III) : 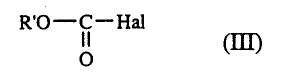
dans laquelle R' représente un groupement alkyle contenant de 1 à 6 atomes de carbone en chaîne droite ou ramifiée, et Hal un atome d'halogène,
pour conduire au composé de formule (IV) : 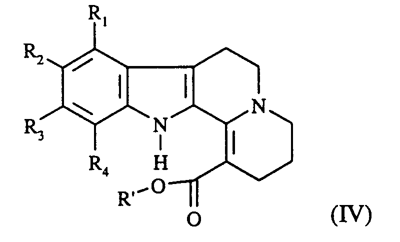
dans laquelle R₁, R₂, R₃, R₄ et R' sont tels que définis précédemment,
qui est ensuite hydrogéné en mélange d'énantiomères *cis* de formule (Va) : 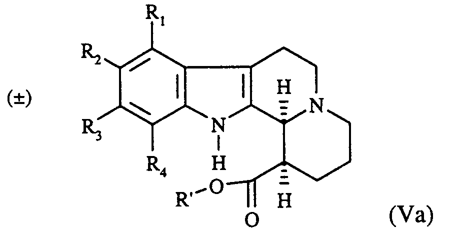
dans laquelle R₁, R₂, R₃, R₄ et R' sont tels que définis précédemment,
par action d'un agent d'hydrogénation, en milieu acide, et en solvant anhydre,
une réaction d'épimérisation pouvant être éventuellement réalisée, à froid, en solvant anhydre, à l'aide d'un hydrure alcalin, afin d'obtenir le mélange d'énantiomères *trans* de formule (Vb) : 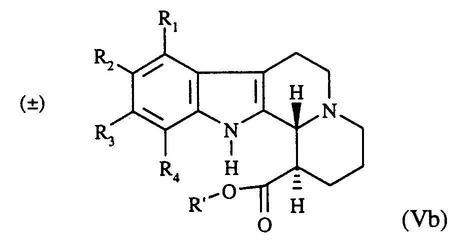
dans laquelle R₁, R₂, R₃, R₄ et R' sont tels que définis précédemment,
l'ensemble des composés de formules (Va) et (Vb) formant l'ensemble des composés de formule (V) : 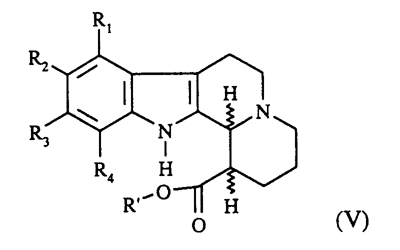
dans laquelle R₁, R₂, R₃, R₄ et R' sont tels que définis précédemment,
composés de formule (V) qui sont à nouveau soumis à hydrogénation, en milieu trifluoroacétique, par le cyanoborohydrure de sodium, pour conduire au composé de formule (VI) : 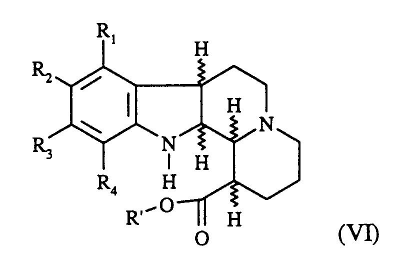
dans laquelle R₁, R₂, R₃, R₄ et R' sont tels que définis précédemment,
qui est soumis :
- soit : A/ à l'action d'un hydrure alcalin, en solvant anhydre, à reflux, pour conduire au composé de formule (Xa) : 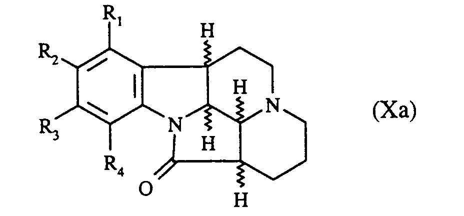
dans laquelle R₁, R₂, R₃ et R₄ sont tels que définis précédemment,
- soit : B/ à l'action de nitrite de sodium, en milieu acide, puis en présence de zinc, pour conduire à l'hydrazine de formule (VII) : 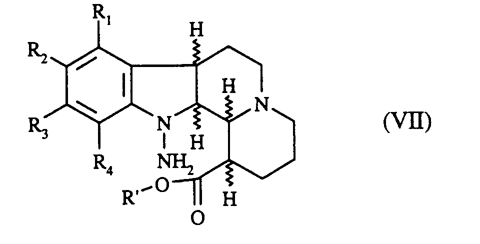
dans laquelle R₁, R₂, R₃, R₄ et R' sont tels que définis précédemment,
qui est ensuite cyclisée, sous l'action d'un hydrure alcalin, en composé de formule (VIII) : 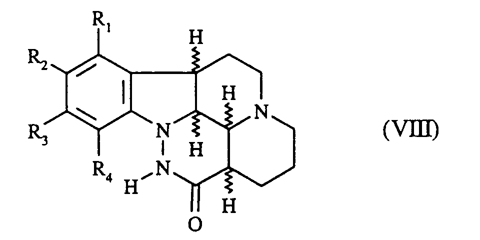
dans laquelle R₁, R₂, R₃ et R₄ sont tels que définis précédemment,
puis soumis, si on le souhaite, à l'action du composé de formule (IX) :
R'₅-Hal (IX)
dans laquelle R'₅ a les mêmes significations que le radical R₅, tel que défini dans la formule (I), l'hydrogène excepté, et Hal représente un atome d'halogène,
après action d'un agent de déprotonation, pour conduire au composé de formule (Xb) : 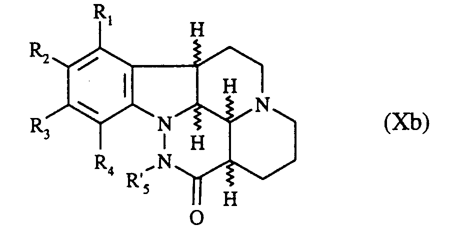
dans lesquelles R₁, R₂, R₃, R₄ et R'₅ sont tels que définis précédemment,
la fonction cétone de l'ensemble des composés de formules (VIII), (Xa) et (Xb) pouvant, si on le souhaite, être réduite, en hydrocarbure correspondant, ou bien soumise à l'action du réactif de Lawesson, afin d'être transformée en la thiocétone correspondante, l'ensemble des composés de formules (VIII), (Xa) et (Xb) ainsi que leurs éventuels produits de réductions ou leurs éventuels dérivés soufrés, formant l'ensemble des composés de formule (I/1) : 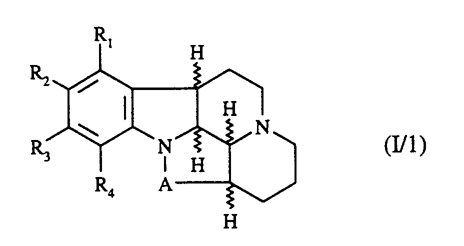
dans laquelle R₁, R₂, R₃, et R₄ sont tels que définis précédemment, et A est tel que défini dans la formule (I),
qui sont :
- soit : soumis à l'action d'un oxydant doux, pour conduire au composé de formule (I/2) : 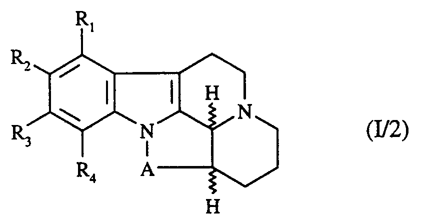
dans laquelle R₁, R₂, R₃, R₄ et A sont tels que définis précédemment,
- soit : soumis à l'action d'un oxydant fort, pour conduire au composé de formule (I/3) : 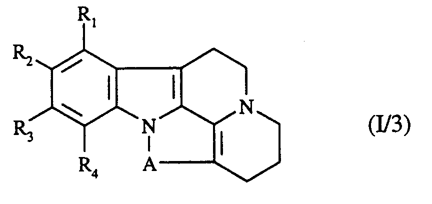
dans laquelle R₁, R₂, R₃, R₄ et A sont tels que définis précédemment,
l'ensemble des composés de formules (I/1), (I/2) et (I/3) formant l'ensemble des composés de formule (I) qui sont, le cas échéant, purifiés par une technique classique de purification, séparés en leurs éventuels énantiomères et diastéréoisomères, et transformés en leurs N-oxydes ou sels d'addition à un acide, pharmaceutiquement acceptables.

8. Compositions pharmaceutiques contenant comme principe actif au moins un composé selon l'une quelconque des revendications 1 à 6, un de ses éventuels N-oxydes, énantiomères, diastéréoisomères ou sels d'addition à un acide, pharmaceutiquement acceptables, seul ou en combinaison avec un ou plusieurs excipients ou véhicules inertes, non toxiques et appropriés.

9. Compositions pharmaceutiques selon la revendication 8 contenant au moins un principe actif, inducteur de tyrosine hydroxylase, selon l'une quelconque des revendications 1 à 6 et utiles dans le traitement des dépressions, de l'anxiété, des troubles de la mémoire au cours de la sénescence et/ou de maladies dégénératives et dans le traitement de la maladie de Parkinson.

## Claims

1. Compounds of the general formula (I): wherein: 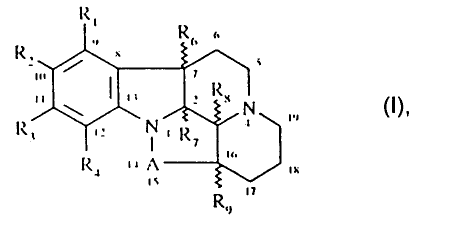
- R₁, R₂, R₃ and R₄, which may be identical or different, are:
* selected independently of one another from:
- a hydrogen atom,
- a halogen atom,
- a hydroxy radical,
- a linear or branched (C₁-C₆)-alkyl radical optionally substituted by one or more halogen atoms, by one or more amino groups, by one or more nitro groups, by one or more linear or branched (C₁-C₆)-alkoxy groups or by one or more aryl radicals, selected from phenyl and naphthyl, which are themselves optionally substituted by one or more halogen atoms, nitro or amino groups or (C₁-C₆)-alkyl or (C₁-C₆)-alkoxy radicals,
- and a linear or branched (C₁-C₆)-alkoxy group optionally substituted by one or more halogen atoms, by one or more amino groups, by one or more nitro groups, or by one or more linear or branched (C₁-C₆)-alkoxy groups,
* or R₁, R₂, R₃ and R₄, taken in pairs and carried by adjacent carbon atoms, form a methylenedioxy or ethylenedioxy group,
- R₆ and R₇:
. either each simultaneously represent hydrogen, adopting a *cis*-configuration with respect to one another,
. or together form a bond,
- R₈ and R₉
. either each simultaneously represent hydrogen, adopting a *cis*- or *trans*-configuration with respect to one another,
. or together form a bond in the case where R₆ and R₇ also together form a double bond,
- 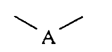 represents a bivalent radical selected from 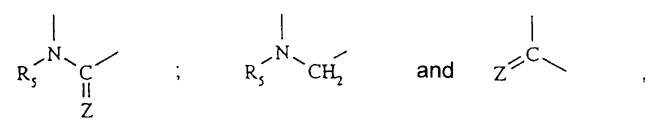
- Z is selected from oxygen and sulphur,
- R₅ is selected from hydrogen and a linear or branched (C₁-C₆)-alkyl group optionally substituted by one or more:
- halogen atoms,
- linear or branched (C₁-C₆)-alkoxy radicals,
- phenyl radicals optionally substituted by one or more halogen atoms, linear or branched (C₁-C₆)-alkyl radicals, or linear or branched (C₁-C₆)-alkoxy radicals, those alkyl and alkoxy radicals optionally being substituted by one or more aryl radicals, selected from phenyl and naphthyl, which are unsubstituted or substituted by one or more groups selected from the halogens and amino, nitro, (C₁-C₆)-alkyl and (C₁-C₆)-alkoxy radicals,
- or radicals 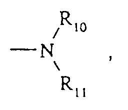 wherein R₁₀ and R₁₁ are selected independently of one another from hydrogen, linear or branched (C₁-C₆)-alkyl radicals and linear or branched (C₁-C₆)-alkoxy radicals,
their possible N-oxides, enantiomers and diastereoisomers and also, where appropriate, their pharmaceutically acceptable acid addition salts.

2. Compound according to claim 1 that is 10-methoxy-2,7,14,15-tetrahydro-14-aza-20,21-dinoreburnamenin-15-one,
its N-oxides, enantiomers and diastereoisomers and pharmaceutically acceptable acid addition salts.

3. Compound according to claim 1 that is 10-chloro-2,7,14,15-tetrahydro-14-aza-20,21-dinoreburnamenin-15-one,
its N-oxides, enantiomers and diastereoisomers and pharmaceutically acceptable acid addition salts.

4. Compound according to claim 1 that is 14,15-dihydro-14-aza-20,21-dinoreburnamenine,
its N-oxides, enantiomers and diastereoisomers and pharmaceutically acceptable acid addition salts.

5. Compound according to claim 1 that is 10,11-methylenedioxy-2,7,14,15-tetrahydro-14-aza-20,21-dinoreburnamenin-15-one,
its N-oxides, enantiomers and diastereoisomers and pharmaceutically acceptable acid addition salts.

6. Compound according to claim 1 that is 9-methoxy-1a,2,3,6,7,7a,12a,12b-octahydro-1*H*,5*H*-benzo[5,6]pyrrolizino[2,1,7-ija]quinolizin-1-one,
its N-oxides, enantiomers and diastereoisomers and pharmaceutically acceptable acid addition salts.

7. Process for the preparation of the compounds of formula (I), characterised in that the compound of formula (II): 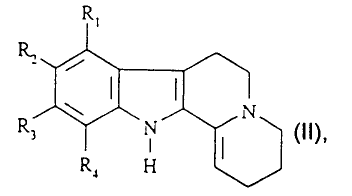
wherein R₁, R₂, R₃ and R₄ are as defined for formula (I),
is subjected to the action of a compound of formula (III): 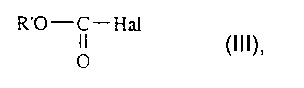
wherein R' represents an alkyl group containing from 1 to 6 carbon atoms in a straight or branched chain, and Hal represents a halogen atom,
to yield a compound of formula (IV): 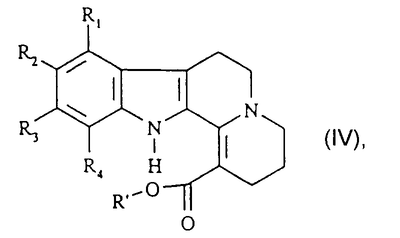
wherein R₁, R₂, R₃, R₄ and R' are as defined above,
which is then hydrogenated to form a mixture of *cis*-enantiomers of formula (Va): 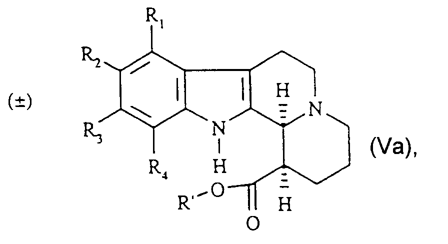
wherein R₁, R₂, R₃, R₄ and R' are as defined above,
by the action of a hydrogenation agent in an acid medium and in an anhydrous solvent,
it optionally being possible for an epimerisation reaction to be effected, at a low temperature, in an anhydrous solvent using an alkali metal hydride in order to obtain a mixture of *trans*-enantiomers of formula (Vb): 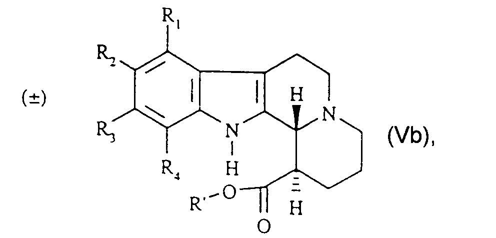
wherein R₁, R₂, R₃, R₄ and R' are as defined above,
the totality of the compounds of formulae (Va) and (Vb) forming the totality of the compounds of formula (V): 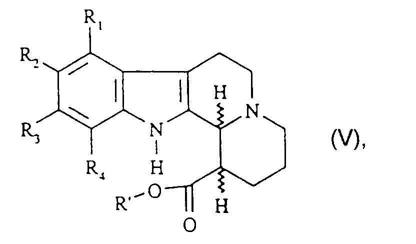
wherein R₁, R₂, R₃, R₄ and R' are as defined above,
which compounds of formula (V) are again subjected to hydrogenation, in a trifluoroacetic acid medium, by means of sodium cyanoborohydride, to yield a compound of formula (VI): 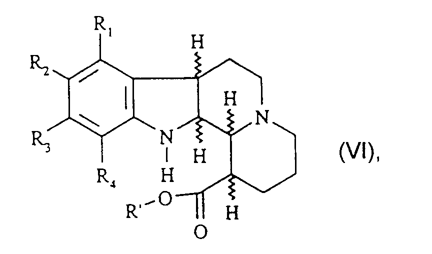
wherein R₁, R₂, R₃, R₄ and R' are as defined above,
which is subjected:
- either: A/ to the action of an alkali metal hydride in an anhydrous solvent, under reflux, to yield a compound of formula (Xa): 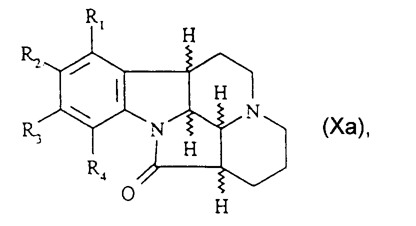
wherein R₁, R₂, R₃ and R₄ are as defined above,
or: B/ to the action of sodium nitrite, in an acid medium, then in the presence of zinc, to yield a hydrazine of formula (VII): 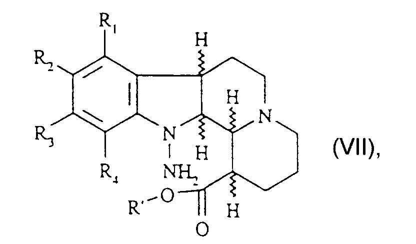
wherein R₁, R₂, R₃, R₄ and R' are as defined above,
which is then cyclised under the action of an alkali metal hydride to form a compound of formula (VIII): 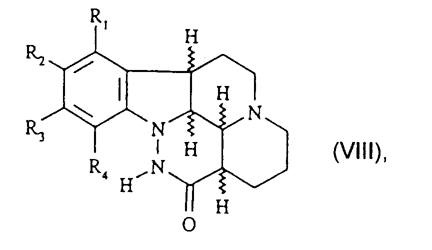
wherein R₁, R₂, R₃ and R₄ are as defined above,
and then subjected, if desired, to the action of a compound of formula (IX):
R'₅-Hal (IX)
,
wherein R'₅ has the same meanings as the radical R₅ as defined for formula (I), with the exception of hydrogen, and Hal represents a halogen atom,
after the action of a deprotonating agent, to yield a compound of formula (Xb): 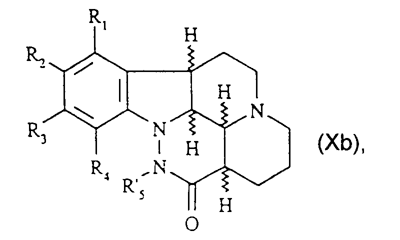
wherein R₁, R₂, R₃, R₄ and R'₅ are as defined above,
it being possible, if desired, for the ketone function of the totality of the compounds of formulae (VIII), (Xa) and (Xb) to be reduced to form the corresponding hydrocarbon, or subjected to the action of Lawesson's reagent, in order to be converted into the corresponding thioketone, the totality of the compounds of formulae (VIII), (Xa) and (Xb), as well as their possible reduction products or their possible thio analogues, forming the totality of the compounds of formula (I/1): 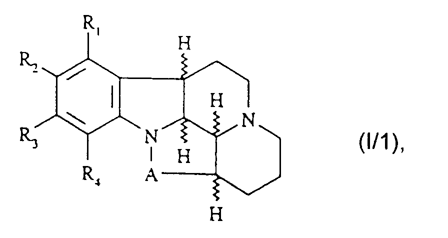
wherein R₁, R₂, R₃ and R₄ are as defined above and A is as defined for formula (I),
which are:
- either: subjected to the action of a gentle oxidising agent to yield a compound of formula (I/2): 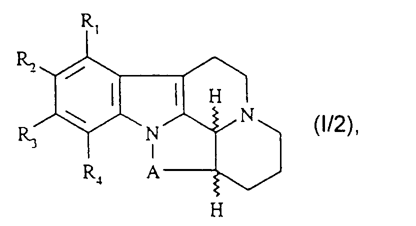
wherein R₁, R₂, R₃, R₄ and A are as defined above,
- or: subjected to the action of a strong oxidising agent to yield a compound of formula (I/3): 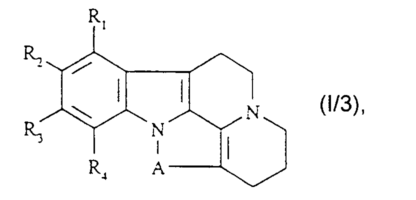
wherein R₁, R₂, R₃, R₄ and A are as defined above,
the totality of the compounds of formulae (I/1), (I/2) and (I/3) forming the totality of the compounds of formula (I) which are, where appropriate, purified by a conventional purification technique, separated into their possible enantiomers and diastereoisomers and converted into their N-oxides or pharmaceutically acceptable acid addition salts.

8. Pharmaceutical compositions that contain as active ingredient at least one compound according to any one of claims 1 to 6, one of its possible N-oxides, enantiomers, diastereoisomers or pharmaceutically acceptable acid addition salts, alone or in combination with one or more suitable inert, non-toxic excipients or carriers.

9. Pharmaceutical compositions according to claim 8 that contain at least one active ingredient, namely an inducer of tyrosine hydroxylase, according to any one of claims 1 to 6 and that can be used in the treatment of depression, anxiety, memory disorders in the course of senescence and/or degenerative diseases and in the treatment of Parkinson's disease.

## Patentansprüche

1. Verbindungen der allgemeinen Formel (I): 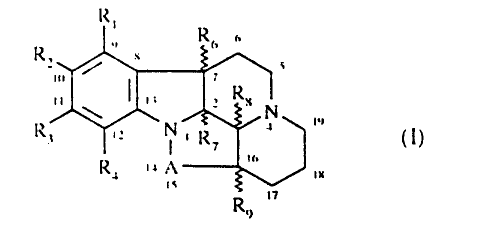 in der:
- R₁, R₂, R₃ und R₄, die gleichartig oder verschieden sein können:
* unabhängig voneinander ausgewählt sind aus:
- einem Wasserstoffatom,
- einem Halogenatom,
- einer Hydroxylgruppe,
- einer geradkettigen oder verzweigten (C₁-C₆)-Alkylgruppe, die gegebenenfalls durch ein oder mehrere Halogenatome oder eine oder mehrere Aminogruppen oder eine oder mehrere Nitrogruppen oder eine oder mehrere geradkettige oder verzweigte (C₁-C₆)-Alkoxygruppen oder durch eine oder mehrere Arylgruppen ausgewählt aus Phenyl und Naphthyl, die ihrerseits gegebenenfalls durch ein oder mehrere Halogenatome, Nitrogruppen, Aminogruppen, (C₁-C₆)-Alkylgruppen oder (C₁-C₆)-Alkoxygruppen substituiert sind, substituiert ist,
- und einer geradkettigen oder verzweigten (C₁-C₆)-Alkoxygruppe, die gegebenenfalls durch ein oder mehrere Halogenatome, eine oder mehrere Aminogruppen, eine oder mehrere Nitrogruppen oder eine oder mehrere geradkettige oder verzweigte (C₁-C₆)-Alkoxygruppen substituiert ist,
* oder R₁, R₂, R₃ und R₄ jeweils zu zweit genommen und von benachbarten Kohlenstoffatomen getragen, gemeinsam eine Methylendioxy- oder Ethylendioxygruppe bilden,
- R₆ und R₇:
. entweder gleichzeitig jeweils Wasserstoff bedeuten, wobei sie in bezug aufeinander eine cis-Konfiguration annehmen,
. oder gemeinsam eine Bindung bilden,
- R₈ und R₉:
. entweder jeweils gleichzeitig Wasserstoff bedeuten und in bezug aufeinander die cis- oder trans-Konfiguration annehmen,
. oder gemeinsam eine Bindung bilden in dem Fall, wo R₆ und R₇ ebenfalls gemeinsam eine Doppelbindung bilden,
- 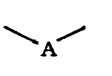 eine zweiwertige Gruppe ist, ausgewählt aus 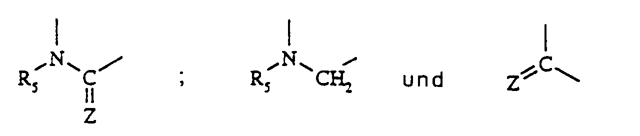 darstellt,
- Z aus Sauerstoff und Schwefel ausgewählt ist,
- R₅ aus Wasserstoff und einer geradkettigen oder verzweigten (C₁-C₆)-Alkylgruppe ausgewählt ist, welche gegebenenfalls substituiert ist durch ein oder mehrere:
- Halogenatome,
- geradkettige oder verzweigte (C₁-C₆)-Alkoxygruppen,
- Phenylgruppen, die gegebenenfalls durch ein oder mehrere Halogenatome, geradkettige oder verzweigte (C₁-C₆)-Alkylgruppen oder geradkettige oder verzweigte (C₁-C₆)-Alkoxygruppen substituiert sind, wobei diese Alkyl- und Alkoxygruppen gegebenenfalls durch eine oder mehrere Arylgruppen ausgewählt aus Phenyl und Naphthyl substituiert sind, die ihrerseits gegebenenfalls durch ein oder mehrere Halogenatome, Aminogruppen, Nitrogruppen, (C₁-C₆)-Alkylgruppen und (C₁-C₆)-Alkoxygruppen substituiert sind,
- oder Gruppen 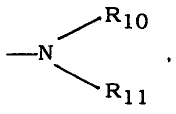 worin R₁₀ und R₁₁ unabhängig voneinander aus Wasserstoff, geradkettigen oder verzweigten (C₁-C₆)-Alkylgruppen und geradkettigen oder verzweigten (C₁-C₆)-Alkoxygruppen ausgewählt sind,
deren mögliche N-Oxide, Enantiomere und Diastereoisomere sowie gegebenenfalls deren Additionssalze mit einer pharmazeutisch annehmbaren Säure.

2. Verbindung nach Anspruch 1, nämlich 10-Methoxy-2,7,14,15-tetrahydro14-aza-20,21-dinoreburnamenin-15-on,
dessen N-Oxide, Enantiomere und Diastereoisomere und dessen Additionssalze mit einer pharmazeutisch annehmbaren Säure.

3. Verbindung nach Anspruch 1, nämlich 10-Chlor-2,7,14,15-tetrahydro-14-aza-20,21-dinoreburnamenin-15-on,
dessen N-Oxide, Enantiomere und Diastereoisomere und dessen Additionssalze mit einer pharmazeutisch annehmbaren Säure.

4. Verbindung nach Anspruch 1, nämlich 14,15-Dihydro-14-aza-20,21-dinoreburnamenin,
dessen N-Oxide, Enantiomere und Diastereoisomere und dessen Additionssalze mit einer pharmazeutisch annehmbaren Säure.

5. Verbindung nach Anspruch 1, nämlich 10,11-Methylendioxy-2,7,14,15-tetrahydro-14-aza-20,21-dinoreburnamenin-15-on,
dessen N-Oxide, Enantiomere und Diastereoisomere und dessen Additionssalze mit einer pharmazeutisch annehmbaren Säure.

6. Verbindung nach Anspruch 1, nämlich 9-Methoxy-1a,2,3,6,7,7a,12a,12b-octahydro-*1H,5H*-benzo[5,6]pyrrolizino[2,1,7-ija]chinolizin-1-on,
dessen N-Oxide, Enantiomere und Diastereoisomere und dessen Additionssalze mit einer pharmazeutisch annehmbaren Säure.

7. Verfahren zur Herstellung der Verbindungen der Formel (I), dadurch gekennzeichnet, daß man die Verbindung der Formel (II): 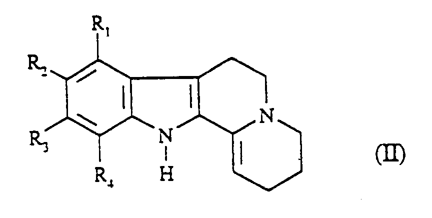
in der R₁, R₂, R₃ und R₄ die bezüglich der Formel (I) angegebenen Bedeutungen besitzen,
der Einwirkung einer Verbindung der Formel (III): 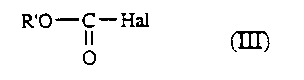
in der R' eine Alkylgruppe mit 1 bis 6 Kohlenstoffatomen in gerader oder verzweigter Kette und Hal ein Halogenatom bedeuten,
unterwirft zur Bildung der Verbindung der Formel (IV): 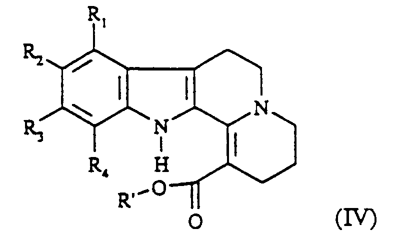
in der R₁, R₂, R₃, R₄ und R' die oben angegebenen Bedeutungen besitzen,
welche anschließend durch Einwirkung eines Hydrierungsmittels in saurem Medium und in einem wasserfreien Lösungsmittel zu einer Mischung der cis-Enantiomeren der Formel (Va) hydriert wird: 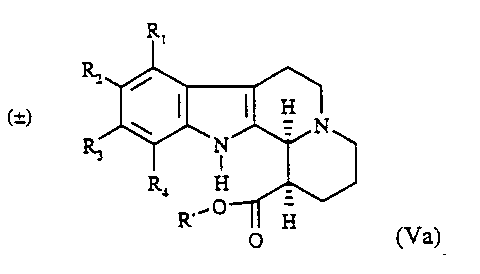
in der R₁, R₂, R₃, R₄ und R' die oben angegebenen Bedeutungen besitzen,
wobei gegebenenfalls eine Epimerisierungsreaktion in der Kälte in einem wasserfreien Lösungsmittel mit Hilfe eines Alkalimetallhydrids durchgeführt werden kann zur Bildung der Mischung der trans-Enantiomeren der Formel (Vb): 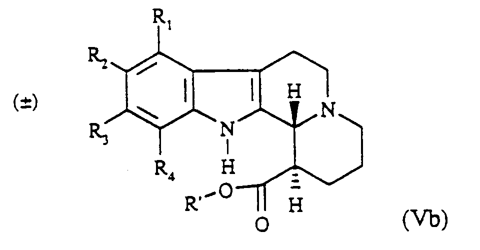
in der R₁, R₂, R₃, R₄ und R' die oben angegebenen Bedeutungen besitzen,
wobei die Gesamtheit der Verbindungen der Formeln (Va) und (Vb) die Gesamtheit der Verbindungen der Formel (V) bildet: 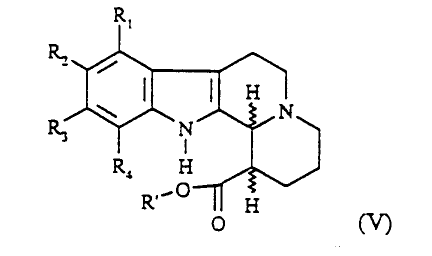
in der R₁, R₂, R₃, R₄ und R' die oben angegebenen Bedeutungen besitzen,
welche Verbindungen der Formel (V) erneut einer Hydrierung in Trifluoressigsäure als Medium mit Natriumcyanborhydrid unterworfen werden zur Bildung der Verbindung der Formel (VI): 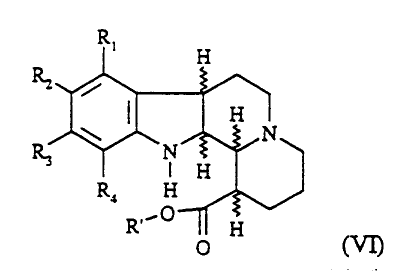
in der R₁, R₂, R₃, R₄ und R' die oben angegebenen Bedeutungen besitzen,
welche dann:
- entweder: A/ der Einwirkung eines Alkalimetallhydrids in wasserfreiem Medium am Rückfluß unterworfen wird zur Bildung der Verbindung der Formel (Xa): 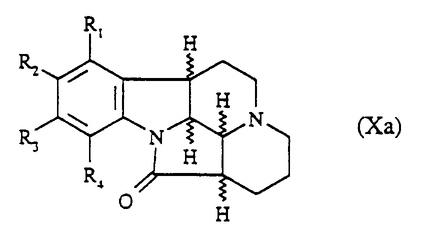
in der R₁, R₂, R₃ und R₄ die oben angegebenen Bedeutungen besitzen,
- oder: B/ der Einwirkung von Natriumnitrit in saurem Medium und dann in Gegenwart von Zink unterworfen wird zur Bildung des Hydrazins der Formel (VII): 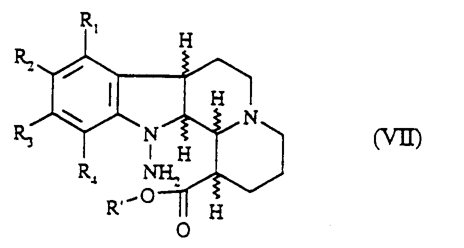
in der R₁, R₂, R₃, R₄ und R' die oben angegebenen Bedeutungen besitzen,
welches anschließend unter Einwirkung eines Alkalimetallhydrids zu der Verbindung der Formel (VIII) cyclisiert wird: 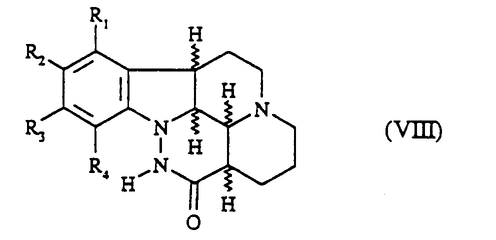
in der R₁, R₂, R₃ und R₄ die oben angegebenen Bedeutungen besitzen,
und dann gewünschtenfalls nach der Einwirkung eines Deprotonierungsmittels der Einwirkung einer Verbindung der Formel (IX) unterworfen wird:
R'₅ - Hal (IX)
in der R'₅ die gleichen Bedeutungen besitzt wie die Gruppe R₅, wie sie bezüglich der Formel (I) definiert worden sind, mit Ausnahme von Wasserstoff, und Hal ein Halogenatom bedeutet,
so daß man die Verbindung der Formel (Xb) erhält: 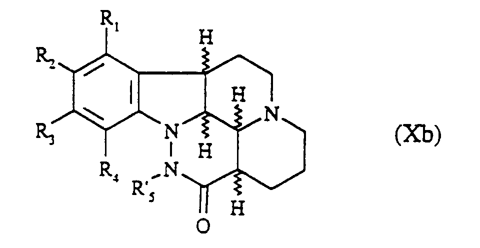
worin R₁, R₂, R₃, R₄ und R'₅ die oben angegebenen Bedeutungen besitzen,
wobei gewünschtenfalls die Keto-Funktion der Gesamtheit der Verbindungen der Formeln (VIII), (Xa) und (Xb) zu dem entsprechenden Kohlenwasserstoff reduziert oder der Einwirkung eines Lawesson-Reagens zur Umwandlung in das entsprechende Thioketon unterzogen werden kann, wobei die Gesamtheit der Verbindungen der Formeln (VIII), (Xa) und (Xb) sowie deren eventuelle Reduktionsprodukte oder deren eventuelle Schwefelderivate die Gesamtheit der Verbindungen der Formel (I/1) bildet: 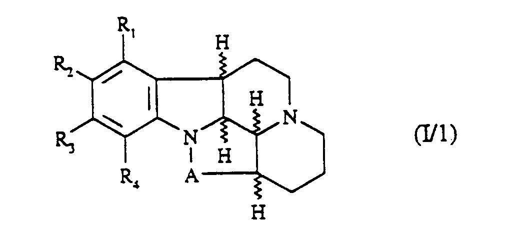
in der R₁, R₂, R₃ und R₄ die oben angegebenen Bedeutungen besitzen und A die bezüglich der Formel (I) angegebenen Bedeutungen besitzt,
welche anschließend:
- entweder: der Einwirkung eines milden Oxidationsmittels unterworfen werden zur Bildung der Verbindung der Formel (I/2): 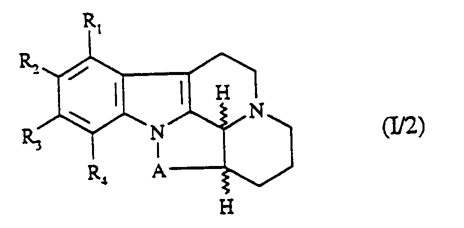
in der R₁, R₂, R₃, R₄ und A die oben angegebenen Bedeutungen besitzen,
- oder: der Einwirkung eines starken Oxidationsmittels unterworfen werden zur Bildung der Verbindung der Formel (I/3): 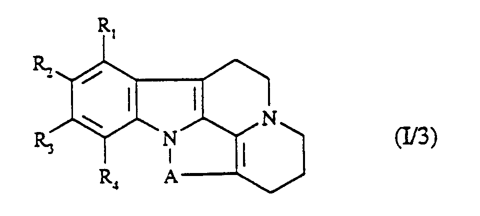
in der R₁, R₂, R₃, R₄ und A die oben angegebenen Bedeutungen besitzen,
wobei die Gesamtheit der Verbindungen der Formeln (I/1), (I/2) und (I/3) die Gesamtheit der Verbindungen der Formel (I) bildet, welche gegebenenfalls mit Hilfe einer klassischen Reinigungsmethode gereinigt, in ihre eventuellen Enantiomeren und Diastereoisomeren aufgetrennt und in ihre N-Oxide oder Additionssalze mit einer pharmazeutisch annehmbaren Säure umgewandelt werden.

8. Pharmazeutische Zubereitungen enthaltend als Wirkstoff mindestens eine Verbindung nach einem der Ansprüche 1 bis 6, eines ihrer eventuellen N-Oxide, Enantiomere, Diastereoisomere oder Additionssalze mit pharmazeutisch annehmbaren Säuren allein oder in Kombination mit einem oder mehreren inerten, nichttoxischen und geeigneten Trägermaterialien oder Bindemitteln.

9. Pharmazeutische Zubereitungen nach Anspruch 8, enthaltend mindestens einen Wirkstoff mit Tyrosin-Hydrolase-Induktionswirkung, nach einem der Ansprüche 1 bis 6 zur Behandlung von Depressionen, der Angst, von Gedächtnisstörungen im Verlaufe des Alterns und/oder von degenerativen Erkrankungen und zur Behandlung der Parkinsonschen Krankheit.
